(19) Europäisches Patentamt
European Patent Office
Office européen des brevets

(11) **EP 3 984 495 B1**

(12) **EUROPÄISCHE PATENTSCHRIFT**

(45) Veröffentlichungstag und Bekanntmachung des Hinweises auf die Patenterteilung:
**21.08.2024 Patentblatt 2024/34**

(21) Anmeldenummer: **20201553.3**

(22) Anmeldetag: **13.10.2020**

(51) Internationale Patentklassifikation (IPC):
**A61C 5/77** (2017.01) **A61C 13/09** (2006.01)

(52) Gemeinsame Patentklassifikation (CPC):
**A61C 5/77; A61C 5/73; A61C 13/0004; A61C 13/08; A61C 13/082; A61C 13/09; B33Y 50/00; B33Y 80/00**

(54) **VERFAHREN ZUM GESTALTEN EINER DENTALEN RESTAURATION**

METHOD FOR FORMING A DENTAL RESTORATION

PROCÉDÉ DE CONCEPTION D'UNE RESTAURATION DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.04.2022 Patentblatt 2022/16**

(73) Patentinhaber: **Ivoclar Vivadent AG**
**9494 Schaan (LI)**

(72) Erfinder:
• **JOHN, Hendrik**
**9470 Buchs (CH)**

• **JUSSEL, Rudolf**
**6805 Feldkirch-Gisingen (AT)**

(74) Vertreter: **Splanemann Patentanwälte Partnerschaft Rumfordstrasse 7 80469 München (DE)**

(56) Entgegenhaltungen:
**EP-A1- 3 530 232    WO-A1-2019/023461 DE-A1- 19 922 870**

**Beschreibung**

Die vorliegende Erfindung betrifft ein Verfahren zum Gestalten einer dentalen Restauration, eine Computervorrichtung zum Gestalten einer dentalen Restauration und ein Computerprogramm für die Computervorrichtung.

Bei der Herstellung von dentalen Restaurationen gibt es oft Probleme bei den Farbnuancen und der Transluzenz, da diese nur schwierig zu bestimmen sind und der Aufbau der Restauration durch verschiedene Schichten und Materialien einen Einfluss auf das endgültige Erscheinungsbild hat. Auch die Farbbestimmung basiert derzeit oft auf der Erfahrung der Zahntechniker oder auf einer Schätzung durch einen subjektiven Farbvergleich mit einem Farbschlüssel. Zunehmend werden heute auch dentale Restaurationen, insbesondere Einzelzahnrestaurationen beim Zahnarzt hergestellt. Da in einigen Fällen der Patient auf die Versorgung in der Zahnarztpraxis wartet, ist eine exakte Auswahl der Farbe bei der Herstellung wichtig.

Die Druckschrift EP 2 486 892 B1 beschreibt beispielsweise, dass ein Erscheinungsbild eines virtuellen Zahns auf einem kalibrierten Bildschirm mit dem Bild des Nachbarzahns durch einen Benutzer verglichen wird, um einen realistischen Eindruck zu gewinnen. Wenn das Ergebnis noch nicht befriedigend ist, lässt sich dann beispielsweise die Schichtdicke oder die Transluzenz anpassen. Bei dem optischen Vergleich durch einen Benutzer auf dem Bildschirm treten jedoch stets Ungenauigkeiten auf.

Die Druckschrift DE 199 22 870 A1 betrifft ein Verfahren zur automatischen individuellen Farb-, Transluzenz-, Helligkeitsund Fluoreszenzgebung von zahntechnischen Restaurationen. In diesem Verfahren wird die dentale Restauration hergestellt. Anschließend wird die dentale Restauration vermessen, um Istwerte für die Farbe, Transluzenz und Helligkeit zu erhalten.

Die Druckschrift WO 2019/023461 A1 betrifft Restaurationen, wie künstliche Zähne, Kappen, Zahnprothesen, Veneers, Brücken, die eine naturgetreuere Farbgebung, Schattierung und Transparenz aufweisen.

Es ist die technische Aufgabe der vorliegenden Erfindung, eine dentale Restauration mit natürlicherem Aussehen und höherer farblicher und geometrischer Genauigkeit herzustellen.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Gestalten einer dentalen Restauration gelöst, mit den Schritten eines Erfassens eines Soll-Datensatzes auf Basis eines natürlichen Zahns, der die optischen Eigenschaften und die Geometrie des natürlichen Zahns wiedergibt; eines Erzeugens eines digitalen Zahnmodells mit einer inneren Architektur; eines Renderns des digitalen Zahnmodells auf Basis der inneren Architektur zum Erzeugen eines Ist-Datensatzes, der die optischen Eigenschaften und die Geometrie des digitalen Zahnmodells wiedergibt; eines Berechnens einer Abweichung zwischen dem Soll-Datensatz und dem Ist-Datensatz; und eines iterativen Veränderns des digitalen Zahnmodells, um eine geringere Abweichung zwischen dem erfassten Soll-Datensatz und dem Ist-Datensatz des erneut gerenderten digitalen Zahnmodells zu erhalten; wobei die Abweichung auf Basis eines euklidischen Abstandes zwischen dem Soll-Datensatz und dem Ist-Datensatz berechnet wird oder die Abweichung auf Basis eines spektralen Abstandes zwischen dem Soll-Datensatz und dem Ist-Datensatz berechnet wird.

Die innere Architektur umfasst den räumlichen Aufbau und die verwendeten Materialien für diesen Aufbau. Da die optischen Eigenschaften der verwendeten Materialien und der räumliche Aufbau bekannt sind, lässt sich aus dem digitalen Zahnmodell ein Ist-Datensatz rendern, der das optische Erscheinungsbild der digitalen Zahnmodells angibt. Durch das automatische Berechnen einer Abweichung zwischen dem Soll-Datensatz und dem Ist-Datensatz und eine daraus abgeleitete Anpassung der inneren Architektur und der verwendeten Materialien kann die dentale Restauration mit einem Aussehen hergestellt werden, das demjenigen des erfassten natürlichen Zahns entspricht. Nach einem Einsetzen des dentalen Restaurationsteils gliedert sich der künstliche Zahn harmonisch bei unterschiedlichsten Lichtsituationen nicht unterscheidbar von den umgebenden natürlichen Zähnen ein. Die Schichtung der dentalen Restauration kann in Abhängigkeit der verwendeten Materialien realitätsgetreu vor der Herstellung simuliert werden, damit der Benutzer exakt das Ergebnis erhält, dass dieser erwartet.

Dabei werden für das Rendering optische Parameter der verwendeten Materialien berücksichtigt, die auf angezeigten Bildern auf einem Bildschirm nicht darstellbar und erkennbar sind. Es können auch bestimmte Materialeigenschaften, die Beeinflussung durch die benachbarten Zähne, das Zahnfleisch und/oder unterschiedliche Lichtsituationen berücksichtigt werden. Das Verfahren ist dadurch von der menschlichen Wahrnehmung unabhängig. Das Verändern des digitalen Zahnmodells kann beispielsweise durch einen Austausch der Materialien mit anderen optischen Eigenschaften oder durch ein Verändern der Anordnung oder Schichtdicke der Materialien in der inneren Architektur erfolgen.

Durch die Berechnung der Abweichung auf Basis eines euklidischen Abstandes zwischen dem Soll-Datensatz und dem Ist-Datensatz wird beispielsweise der technische Vorteil erreicht, dass sich mit geringem Aufwand eine genaues Abweichungsmaß ermitteln lässt. Wenn die Werte des Soll-Datensatzes in besonderen Farbwerten, wie beispielsweise z.B. in Lab-Werten, und nicht als Zahnfarben eines Zahnfarbschlüssels vorliegen, kann der Vergleich mit hoher Präzision auf Basis eines Vergleichs der Lab-Istwerte mit den Lab-Sollwerten erfolgen.

Durch die Berechnung der Abweichung auf Basis eines spektralen Abstandes zwischen dem Soll-Datensatz und dem Ist-Datensatz wird beispielsweise der technische Vorteil erreicht, dass ein genauer Farbabgleich zwischen dem Solldatensatz und dem Ist-Datensatz stattfindet.

In einer weiteren technisch vorteilhaften Ausführungsform des Verfahrens umfasst der Soll-Datensatz Daten über ein zweidimensionales oder dreidimensionales Abbild des natürlichen Zahns, Daten über einen Aufnahmewinkel zum Zahn, Daten über eine Farbinformation des Zahns, Daten über ein Farbspektrum des Zahns und/oder Daten über eine Geometrieinformation des Zahns. Dadurch wird beispielsweise der technische Vorteil erreicht, dass besonders geeignete Daten zur Rekonstruktion und Berechnung der Abweichung verwendet werden.

In einer weiteren technisch vorteilhaften Ausführungsform des Verfahrens wird der Soll-Datensatz durch eine digitale Kamera, einen 3D-Scanner, ein 3D-Kamerasystem, ein Spektrometer oder einen digitalisierten Farbschlüssel erfasst. Dadurch wird beispielsweise der technische Vorteil erreicht, dass der Soll-Datensatz auf einfache und schnelle Weise erhalten werden kann, ohne dabei die Umgebungsbedingungen berücksichtigen zu müssen, da diese im Anschluss durch das Computerprogramm herausgerechnet werden können.

In einer weiteren technisch vorteilhaften Ausführungsform des Verfahrens umfasst der Soll-Datensatz optische Eigenschaften eines Restzahns. Die Daten des Restzahns können in diesem Fall ebenfalls erfasst werden. Die Daten umfassen ebenfalls die optischen Eigenschaften und/oder die Geometrie des Restzahns. Dadurch wird beispielsweise der technische Vorteil erreicht, dass eine präzise und genaue Anpassung der dentalen Restauration auf Basis der verbleibenden Zahnsubstanz erhalten wird.

In einer weiteren technisch vorteilhaften Ausführungsform des Verfahrens wird das digitale Zahnmodell auf Basis der optischen Eigenschaften des Restzahns, der Geometrie des Restzahns, der umgebenden Nachbarzähne, der Mundsituation, der Berücksichtigung der Gingiva oder Zahnfleischfarbe und/oder des verwendeten Klebematerials zur Fixierung der dentalen Restauration auf dem Restzahn gerendert. Das Rendern wird in Verbindung mit den optischen Eigenschaften der dentalen Restauration durchgeführt. Dadurch wird beispielsweise ebenfalls der technische Vorteil erreicht, dass eine präzise und genaue Anpassung der dentalen Restauration auf Basis der verbleibenden Zahnsubstanz erhalten wird.

In einer weiteren technisch vorteilhaften Ausführungsform des Verfahrens der Schritt des Erzeugens des digitalen Zahnmodells den Schritt eines Importierens, Berechnens und/oder Festlegens eines Zahnmodells mit einer vorgegebenen inneren Architektur.

Der Schritt des Importierens kann dem Schritt des Erzeugens vorangestellt werden. Dadurch wird beispielsweise der technische Vorteil erreicht, dass das Verfahren beschleunigt wird und aus einer Anzahl vorhandener digitaler Zahnmodele ausgewählt werden kann.

In einer weiteren technisch vorteilhaften Ausführungsform des Verfahrens wird das digitale Zahnmodell in einem Winkel gerendert, der dem Aufnahmewinkel beim Erfassen des Zahns oder/und einer vorgegebenen Lichtsituation entspricht. Dadurch wird beispielsweise der technische Vorteil erreicht, dass ein besserer Vergleich zwischen dem Ist-Datensatz und dem Soll-Datensatz durchgeführt werden kann und der Rechenaufwand zum Rendern der dentalen Restauration auf dem Restzahn oder der Präparation auf die entsprechende Perspektive reduziert und somit beschleunigt wird.

In einer weiteren technisch vorteilhaften Ausführungsform des Verfahrens wird das digitale Zahnmodell durch Änderung einer Materialzuordnung zu einem Subvolumen verändert. Dadurch wird beispielsweise der technische Vorteil erreicht, dass größere Abweichungen ausgeglichen werden können und dadurch ein natürlicher Aufbau und ein natürliches Aussehen der dentalen Restauration erreicht wird.

In einer weiteren technisch vorteilhaften Ausführungsform des Verfahrens wird das digitale Zahnmodell durch eine Änderung der Subvolumen unter Beibehaltung der äußeren Geometrie verändert. Dadurch wird beispielsweise ebenfalls der technische Vorteil erreicht, dass kleinere Abweichungen ausgeglichen werden können, ohne die Materialien auszutauschen und dadurch ein natürlicher Aufbau und ein natürliches Aussehen der dentalen Restauration erreicht wird.

In einer weiteren technischen vorteilhaften Ausführungsform des Verfahrens wird ein Ausgleich zwischen funktionalen Anforderungen, wie beispielsweise vorgegeben Mindestwandstärken und ästhetischen Anforderungen bestimmt und optional dem Anwender zur Auswertung vorgeschlagen.

In einer weiteren technisch vorteilhaften Ausführungsform des Verfahrens wird die dentale Restauration auf Basis des Ist-Datensatzes hergestellt. Dadurch wird beispielsweise ebenfalls der technische Vorteil erreicht, dass sich die dentale Restauration automatisch herstellen lässt und durch die vorherige Simulation eine korrekte Farbwiedergabe gegeben ist, die dem Soll-Datensatz entspricht.

In einer weiteren technisch vorteilhaften Ausführungsform des Verfahrens erfolgt die Herstellung der dentalen Restauration mittels einer Multimaterial-3D-Druckeinrichtung oder einer Fräseinrichtung. Das Herstellen der dentalen Restauration kann auch die Schritte eines Druckens der dentalen Restauration in einem Stereolithografieverfahren und ein anschließendes Sintern umfassen. Bei der Presstechnik unter Verwendung von Rohlingen mit Farbverlauf kann die Orientierung und Position der Restauration im Rohling individuell und gezielt festgelegt werden. Durch die beiden letztgenannten Herstellungsverfahren werden beispielsweise die technischen Vorteile erreicht, dass sich die dentale Restauration auf schnelle Weise mit einem natürlichen Aussehen herstellen lässt.

In einer weiteren technisch vorteilhaften Ausführungsform des Verfahrens erfolgt die Herstellung der dentalen Restauration mittels eines Additive-Manufacturing-Verfahrens aus multiplen oder selektiv eingefärbten Materialien. Das Herstellen der dentalen Restauration kann durch die Schritte eines additiven Fertigungsverfahrens umgesetzt werden, bei dem multiple Materialien den vorgegebenen Subvolumen der dentalen Restauration zugeordnet, selektiv im Raum aufgetragen werden, oder monochromatische Materialien schichtweise aufgetragen werden, wobei einzelne Schichten selektiv eingefärbt oder verfärbt werden. Durch diese additiven Herstellungsverfahren werden beispielsweise die technischen Vorteile erreicht, dass sich die dentale Restauration nach den Vorgaben des digitalen Soll-Datensatzes auf schnelle Weise mit einem natürlichen Aussehen herstellen lässt.

Gemäß einem zweiten Aspekt wird die technische Aufgabe durch eine Computervorrichtung zum Gestalten einer dentalen Restauration, mit einem Sensor zum Erfassen eines Soll-Datensatzes auf Basis eines natürlichen Zahns gelöst, die geeignet ist, das Verfahren nach dem ersten Aspekt auszuführen. Dadurch werden die gleichen technischen Vorteile wie durch das Verfahren nach dem ersten Aspekt erreicht.

Gemäß einem dritten Aspekt wird die technische Aufgabe durch ein Computerprogramm gelöst, das Befehle umfasst, die bewirken, dass die Computervorrichtung nach dem zweiten Aspekt die Verfahrensschritte nach dem ersten Aspekt ausführt.

Ausführungsbeispiele der Erfindung sind in den Zeichnungen dargestellt und werden im Folgenden näher beschrieben.

Es zeigen:

Fig. 1    eine schematische Ansicht eine Computervorrichtung zum Gestalten einer dentalen Restauration;

Fig. 2    eine schematische Darstellung einer Abweichung zwischen einem Soll-Datensatz und einem Ist-Datensatz;

Fig.3    eine schematische Darstellung zur Berechnung einer Abweichung zwischen einem Soll-Datensatz und einem Ist-Datensatz; und

Fig. 4    ein Blockdiagram eines Verfahrens zum Gestalten einer dentalen Restauration.

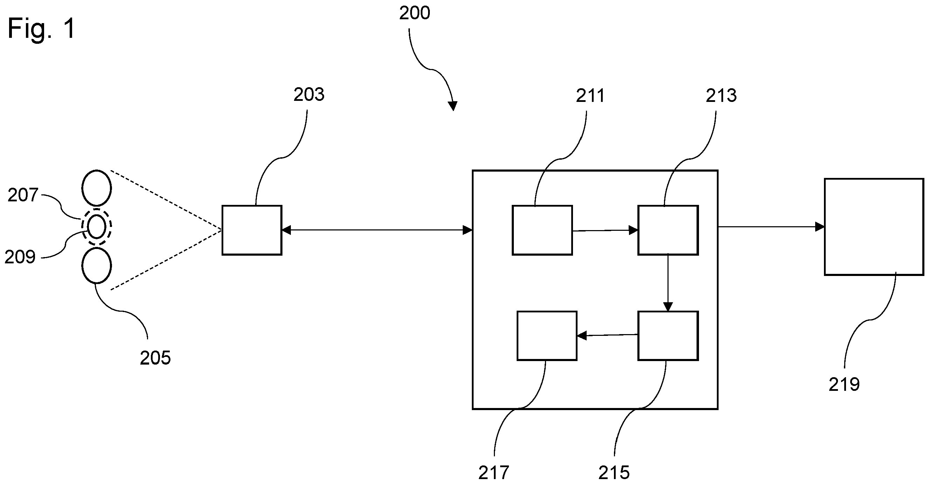

Fig. 1 zeigt eine schematische Ansicht eine Computervorrichtung 200 zum Gestalten einer dentalen Restauration 207. Die Computervorrichtung 200 umfasst einen Sensor 203 zum Erfassen eines Soll-Datensatzes auf Basis eines natürlichen Zahns 205. Der Sensor 203 ist in der Lage, Daten über einen Aufnahmewinkel zum Zahn 205, Daten über eine Farbinformation des Zahns 205, Daten über ein Farbspektrum des Zahns 205, Daten über eine Transluzenz des Zahns 205 und/oder Daten über eine Geometrieinformation des Zahns 205 zu erfassen. Der Sensor 203 liefert dabei die Daten, die in einen Soll-Datensatz für die spätere Berechnung eingebettet werden.

Bei dem Sensor 203 handelt es sich beispielsweise um eine elektronische digitale Kamera, einen 3D-Scanner mit Farberfassung oder eine TOF-Kamera, die mit einem Laufzeitverfahren (TOF - Time of Flight, TOF) oder eine Kamera, die mittels Streifenlicht und Triangulation Abstände messen kann. Durch den Sensor 203 werden die Farb- und Geometrieinformation des Zahns 205 erfasst, wie beispielsweise eine Farbverteilung, eine Transluzenz und/oder eine Form des Zahnes 205. Der Sensor 203 für eine Farbnahme kann ein anderer sein als für die Aufnahme der Geometrie. Beispielsweise kann die Farbnahme durch eine Kamera erfolgen und die Geometrie durch einen separaten Scanner erfasst werden. Es kann aber auch ein analoger Abdruck erzeugt werden, der anschließend mittels eines Lab-Scanners digitalisiert wird.

Im Allgemeinen kann als Sensor 203 jede Vorrichtung verwendet werden, die geeignet ist, Farb-, Transluzenz- und/oder Geometrieinformation des Zahnes 205 zu erfassen, die als spätere Basis für den Soll-Datensatz dient.

Durch den Sensor 203 lässt sich beispielsweise die Geometrie- und Farbinformation auf Basis eines Nachbarzahns 205 erfassen, die dann als Vorlage für die Herstellung der benachbarten dentalen Restauration 207 dient. Zudem kann der Sensor 203 ausgebildet sein, einen Aufnahmewinkel der Perspektive in Bezug zum Zahn 203 zu bestimmen. Der Sensor 203 kann ein Teil eines mobilen Gerätes sein, wie beispielsweise eines Smart Phone oder Tablets. Durch den Sensor 205 lässt sich eine Farbmessung oder Transluzenz-Messung der Nachbarzähne 207 beispielsweise basierend auf den acht natürlichen Farbräumen durchführen. Zusätzlich können über den Sensor 203 die optischen Eigenschaften und die Geometrie eines Restzahns 209, wie beispielsweise eines Zahnstumpfes, erfasst werden, auf dem die dentale Restauration 207 aufgesetzt werden soll.

Die Computervorrichtung 200 umfasst weiter eine Erzeugungseinheit 211 zum Erzeugen eines digitalen Zahnmodells mit einer inneren Architektur. Die innere Architektur spiegelt einen inneren Aufbau des digitalen Zahnmodells wider. Die innere Architektur kann eine Zusammensetzung aus mindestens zwei dentalen Restaurationsmaterialien mit unterschiedlichen optischen und/oder physikalischen Eigenschaften umfassen.

Diese unterschiedlichen dentalen Restaurationsmaterialien können im Falle von additiven Fertigungsverfahren in Voxeln angeordnet sein oder im Falle eines graduierten Werkstoffes in Schichten. Bei monochromatischen Werkstoffen können die Schichten selektiv eingefärbt werden. Die innere Architektur des Zahnmodells umfasst nicht nur dreidimensionale Informationen, sondern auch Informationen über die physikalisch optischen Eigenschaften der Restaurationsmaterialien, wie beispielsweise einen Absorptionskoeffizient oder einen Streukoeffizient der verwendeten Materialien.

Das digitale Zahnmodell kann aus einer Architektur mit unterschiedlichen Subvolumen (Shells) aufgebaut sein, denen die jeweiligen optischen und/oder physikalischen Materialkennwerte zugeordnet sind. Zu diesem Zweck werden die verwendeten Materialien und Halbzeuge im Vorfeld optisch charakterisiert und parametrisiert.

Durch die Erzeugungseinheit 211 kann beispielsweise sowohl die äußere Form des digitalen Zahnmodells als auch die innere Architektur in einem CAD-Prozess modelliert werden. Das digitale Zahnmodell des zu ersetzenden Zahns kann hierzu anfänglich als dreidimensionales Zahnmodell mit vorgegebener innerer Architektur aus einer digitalen Zahn-Bibliothek importiert werden, um anschließend an die gegebene Patientensituation angepasst zu werden. Bei dem dreidimensionalen Zahnmodell geht man von einem idealisierten dreidimensionalen Aufbau aus, bei dem das Erscheinungsbild bereits bekannt ist, also bereits vorberechnet wurde. Über eine automatische Zuordnung des Soll-Datensatzes zu einem definierten Farbraum wird vom System bereits eine Vorauswahl für eine Materialkombination für den IST-Datensatz der Restauration getroffen und dem Anwender vorgeschlagen.

Die Computervorrichtung 200 weist weiter eine Rendering-Einheit 213 auf, die das optische Erscheinungsbild, d.h. die Ist-Situation, des biomimetischen Zahnersatzes auf Basis des erzeugten digitalen Zahnmodells optisch simuliert. Bei der Simulation werden nicht nur die innere Architektur des Zahnes, sondern auch die optischen Eigenschaften der verwendeten Materialien und gegebenenfalls die mechanischen Eigenschaften berücksichtigt.

Zu diesem Zweck werden neben den dreidimensionalen Geometrieinformationen die optischen Parameter und/oder physikalischen Eigenschaften für alle Schichten oder Subvolumen unterschiedlicher Materialien des digitalen Zahnmodells in die Simulationssoftware der Rendering-Einheit 213 importiert. Das Rendern des digitalen Zahnmodells kann auch unter Berücksichtigung der optischen Eigenschaften eines Restzahnes 205 und des verwendeten Klebematerials zur Fixierung der dentalen Restauration 207, der Nachbarzähne, der Zahnfleischfarbe und/oder der Mundsituation erfolgen.

Ist der Aufnahmewinkel des Soll-Datensatzes bekannt, kann das Rendering des digitalen Zahnmodells aus einer Perspektive durchgeführt werden, die derjenigen der erfassten Aufnahme entspricht. Dadurch kann ein zweidimensionales Bild von dem gerenderten digitalen Zahnmodell als Ist-Datensatz aus der Perspektive erzeugt werden, die der Perspektive beim Erfassen des Soll-Datensatzes entspricht. Durch eine Überlagerung der zweidimensionalen Bilder des Soll- und Ist-Datensatzes mit gleicher Perspektive kann eine Abweichung zwischen Soll -und Ist-Datensatz berechnet werden.

Durch das Rendering wird das optische Erscheinungsbild der dentalen Restauration 207 ggf. unter Berücksichtigung des Restzahns und Klebematerials simuliert und ein Ist-Datensatz erzeugt, der die gleichen Parameter wie der Soll-Datensatz umfasst, um eine Abweichung berechnen zu können. Zusätzlich weist die Computervorrichtung 200 eine Berechnungseinheit 215 auf, die eine numerische Abweichung $\Delta E_{S,I}$ zwischen dem Soll-Datensatz und dem Ist-Datensatz berechnet. Die Abweichung $\Delta E_{S,I}$ gibt einen Wert an, der den Unterschied zwischen dem Soll-Datensatz und dem Ist-Datensatz quantifiziert. Dabei kann beispielsweise ein Map-Matching des Ist-Datensatzes mit dem Farb- und Transluzenz-Schema des Soll-Datensatzes durchgeführt werden, so dass die Abweichung $\Delta E_{S,I}$ aus einem Differenzbild erhalten wird.

Die Abweichung $\Delta E_{S,I}$ kann auf Basis eines euklidischen Abstandes oder eines spektralen Abstandes zwischen dem Soll-Datensatz und dem Ist-Datensatz berechnet werden.

Zusätzlich umfasst die Computervorrichtung 200 eine Veränderungseinheit 217 zum automatischen und iterativen Verändern des digitalen Zahnmodells, um eine geringere Abweichung $\Delta E_{S,I}$ zwischen dem erfassten Soll-Datensatz und dem Ist-Datensatz des erneut gerenderten digitalen Zahnmodells zu erhalten. Die Veränderungseinheit 217 kann zu diesem Zweck beispielsweise autonom eine Schichtdicke oder Materialzuordnung des Zahnmodells verändern, so dass sich die optischen Eigenschaften dem Soll-Datensatz annähern, so dass das reale Erscheinungsbild erhalten wird.

Es erfolgt beispielsweise eine iterative Veränderung des digitalen Zahnmodells durch Änderung der Materialzuordnung zu den Subvolumen unter Beibehaltung der äußeren Geometrie des Zahns, beispielsweise zwischen einem Schneide- und Dentinbereich. Dabei werden die Materialien und optischen Parameter einem modifizierten dreidimensionalen Zahnmodell einschließlich Präparation und Zementschicht zugeordnet und erneut gerendert und das Ergebnis mit dem Soll-Datensatz verglichen und eine Abweichung $\Delta E_{S,I}$ zwischen dem Ist-Datensatz und dem Soll-Datensatz berechnet. Die verwendbaren Dentalmaterialien können in einer ganzen Farbpalette und unterschiedlichen Transluzenzstufen vorliegen.

Aus dem veränderten Zahnmodell wird erneut ein Ist-Datensatz gerendert und eine Abweichung $\Delta E_{S,I}$ zwischen dem Ist-Datensatz und dem Soll-Datensatz berechnet.

Liegt die berechnete Abweichung $\Delta E_{S,I}$ beispielsweise über einem vorgegebenen Wert kann das Material oder die Materialkombination für die dentale Restauration 207 unter Beibehaltung der äußeren Form angepasst werden. Liegt die berechnete Abweichung $\Delta E_{S,I}$ unter einem vorgegebenen Wert, erfolgt die Feinabstimmung über die Variation der Subvolumen der inneren Architektur, bzw. die topographische Veränderung der Grenzfläche zwischen Schneide und Dentin unter Beibehaltung der äußeren Geometrie, wobei durch die topographischen Veränderung der Grenzfläche auch eine Charakterisierung der Restauration erfolgen kann.

Ist ein vorgegebener unterer Wert für die Abweichung $\Delta E_{S,I}$ erreicht, wird die Gestaltung der Restauration 207 und die Materialauswahl des digitalen Zahnmodells abgeschlossen, so dass die dentale Restauration 207 gefertigt werden kann. Liegt die Abweichung $\Delta E_{S,I}$ unter dem vorgegebenen Wert beschreibt das digitale Zahnmodell somit die finale dentale Restauration 207.

Statt mit Grenzwerten zu arbeiten, können auch die beide Anpassungsmethoden der Materialanpassung und der Grenzflächenanpassung gleichwertig nebeneinander angewendet werden.

Diese kann mittels 3D-Druck mit mehreren Materialien oder mittels Fräsen aus einem Rohling durch eine automatische Herstellungseinrichtung 219 hergestellt werden. Nach einem Einsetzen der dentalen Restauration 207 in den Mund des Patienten ist das optische Erscheinungsbild des biomimetischen Zahnersatzes optimal an das natürliche Erscheinungsbild angenähert.

Durch die Computervorrichtung 200 kann die dentale Restauration 207 so naturgetreu (biomimetisch) wie möglich hergestellt werden und optisch so gut wie möglich in die Mundraumumgebung des Patienten integriert werden.

Die farbliche und ästhetische Abschätzung und Auswahl für die eigesetzten Materialien und Farben erfolgt nicht subjektiv durch einen Benutzer, sondern objektiv wiederholbar durch einen kalibrierten automatischen Prozess, der stets nach denselben Regeln durchgeführt wird. Das optische Zusammenspiel der verwendeten Materialien, Präparation, Zementschicht, Dentin und Schneide und der Mundsituation (Rot-Weiß-Ästhetik) kann realitätsgetreu simuliert und gerendert werden, da die optischen Parameter der verwendeten Materialien dezidiert vorliegen.

Dadurch wird ein Soll-/Ist-Vergleich ermöglicht, mit dem der innere Aufbau oder die innere Architektur der dentalen Restauration 207 iterativ angepasst werden kann. Dem Benutzer können durch das Rendern entsprechende Farben, Materialien, Halbzeuge und/oder Herstellverfahren vorgegeben werden, um ein ästhetisch optimales, biomimetisches Ergebnis für die dentale Restauration 207 zu erzielen. Auf diese Weise wird eine dentale Restauration 207 erhalten, die sich optimal in die bestehende Mundraum-Situation des Patienten einfügt. Die gefertigte und applizierte dentale Restauration 207 ist ästhetisch optimal in das Gesamtbild integriert, ohne dass es auf eine subjektive Erfahrung eines Benutzers ankommt, der das Material oder Halbzeug nach einem Farbschlüssel auswählt. Ein Erfahrungswissen kann in einer Software berücksichtigt oder integriert werden, so dass auch nicht-erfahrene Zahntechniker davon profitieren können. Dies betrifft beispielsweise die Gestaltung der inneren Architektur des Zahnes oder eine Maltechnik. Es könnte dem Benutzer auch ermöglicht werden, persönliche Präferenzen zur Auswahl anzubieten (z.B. gemalt nach einem spezifizierten Schema) oder demographische Unterschiede zu berücksichtigen (Glanzgrad).

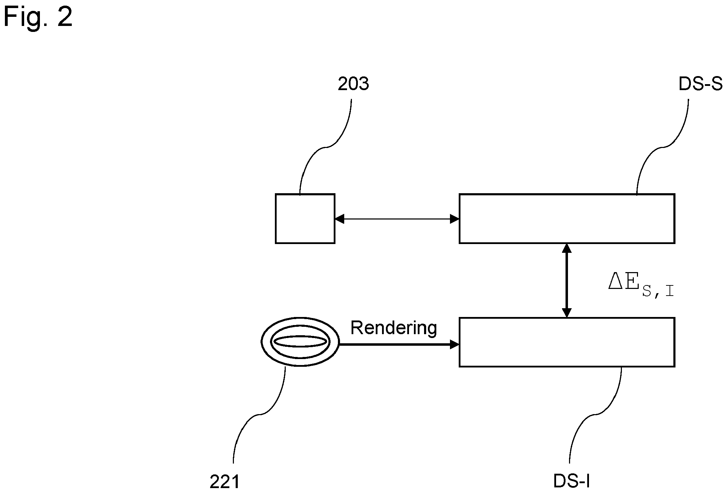

Fig. 2 zeigt eine schematische Darstellung einer Abweichung $\Delta E_{S,I}$ zwischen dem Soll-Datensatz DS-S und dem Ist-Datensatz DS-I. Der Soll-Datensatz DS-S wird auf Basis einer Messung des Zahns 205 durch den Sensor 203 erhalten. Das digital erzeugte Zahnmodell 221 umfasst Daten über die räumliche Geometrie der dentalen Restauration 205 und die zugeordneten Materialien, aus denen die dentale Restauration 207 hergestellt werden soll. Die optischen und physikalischen Eigenschaften, die für das Rendering erforderlich sind, sind in der Rendering-Software bekannt. Diese können aus einer Parametertabelle entnommen werden, die ständig um neue Materialien ergänzt wird.

Der Ist-Datensatz DS-I wird aus dem Zahnmodell 221 erhalten, indem das Zahnmodell 221 einem Rendering unterzogen wird, bei dem die räumliche Geometrie der dentalen Restauration 205 und die optischen und physikalischen Eigenschaften der verschiedenen, vorkommenden Materialien, ggf. einschließlich Klebematerial und Stumpf, berücksichtigt werden.

Die Ausbreitung von Licht in dem Zahnmodell 221 kann durch die Maxwell-Gleichungen beschrieben werden. Das Rendering verwendet beispielsweise die Strahlungstransportgleichung (RTE - Radiative Transport Equation), bei der ein Ausbreitungsmedium durch den Absorptionskoeffizienten, den Streukoeffizienten, den Brechungsindex und die Streuphasenfunktion beschrieben wird. Das digitale Zahnmodell 221 umfasst beispielsweise die Daten über die räumliche Geometrie der dentalen Restauration 205 und die innere Architektur sowie den Absorptionskoeffizienten, den Streukoeffizienten, den Brechungsindex und die Streuphasenfunktion für die jeweiligen Materialien der Architektur.

Die Streuphasenfunktion kann beim Rendern auf Basis des Zahnmodells 205 mit den genannten Parametern in einer Monte-Carlo-Simulation (Ray Tracing) in jeder gewünschten Genauigkeit numerisch gelöst werden, bei der eine Vielzahl von Photonen auf zufälligen Pfaden durch das Zahnmodell propagieren. Daraus lässt sich durch das Rendern ein Ist-Datensatz DS-I für das Aussehen der dentalen Restauration 205 errechnen, der die verwendeten Materialien, die äußere Form und die innere Architektur des Zahnmodells 221 berücksichtigt. Dieser berechnete Ist-Datensatz DS-I kann anschließend mit dem Soll-Datensatz DS-S, der auf Basis eines Nachbarzahns gewonnen worden ist, verglichen werden. Zur Vereinfachung des Vergleichs kann dieser in einer zweidimensionalen Ableitung (zweidimensionales Bild) erfolgen. Als Maß für eine Abweichung $\Delta E_{S,I}$ zwischen dem Ist-Datensatz und dem Soll-Datensatz wird ein nummerischer Wert berechnet. Anschließend wird das digitale Zahnmodell solange verändert, bis die Abweichung $\Delta E_{S,I}$ minimal oder unter einem vorgegebenen Schwellwert ist.

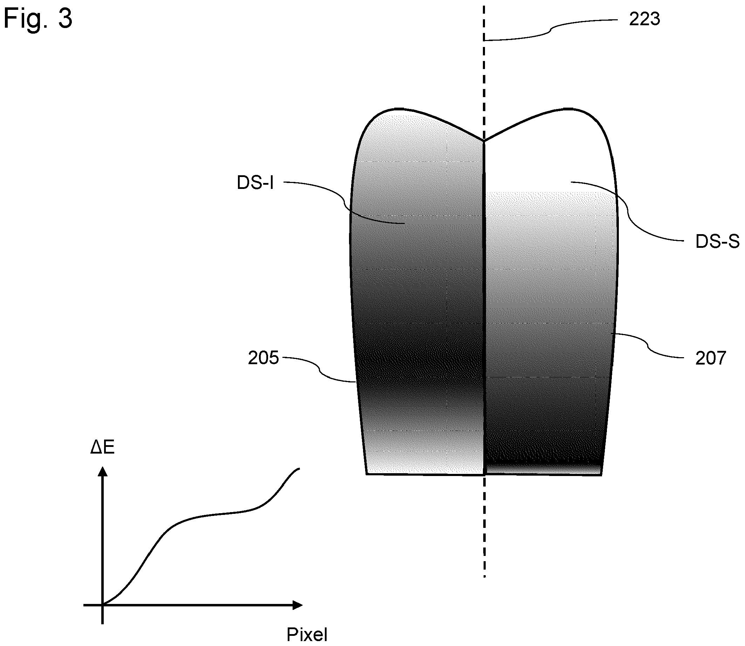

Fig.3 zeigt eine schematische Darstellung zur Berechnung einer Abweichung $\Delta E_{S,I}$ zwischen dem Soll-Datensatz DS-S und einem Ist-Datensatz DS-I. Beispielsweise umfasst der Datensatz DS-S eine Darstellung des Zahns 205 in einer vorgegebenen Perspektive. Demgegenüber umfasst der Datensatz DS-I, der aus dem Zahnmodell gerendert worden ist, eine Darstellung des Zahnmodells in der gleichen Perspektive. Die beiden Abbildungen aus dem Soll-Datensatz DS-S und dem Ist-Datensatz DS-I werden auf die gleiche Größe skaliert und nebeneinandergesetzt. Die euklidische Abweichung $\Delta E_{S,I}$ zwischen dem Soll-Datensatz DS-S und dem Ist-Datensatz DS-I kann berechnet werden, indem die Unterschiede in den Farbwerten der Pixel entlang der Vergleichslinien 223 aufsummiert werden, beispielsweise im L*a*b*-Farbraum. Diese Vergleichslinien 223 können beliebig verschoben werden, indem beispielsweise die Zahnhälften zusammengeschoben werden. Im Allgemeinen kann die Vergleichslinie 223 jedoch auch einen anderen Verlauf aufweisen. Der Vergleich kann auf Pixel-Ebene als kleinste Auflösung erfolgen.

$$\Delta E_{S,I} = \sqrt{(L_S^* - L_I^*)^2 + (a_S^* - a_I^*)^2 + (b_S^* - b_I^*)^2}$$

Je größer der Unterschied im Farbverlauf entlang der Vergleichslinie 223 ist, desto größer ist die numerische Abweichung $\Delta E_{S,I}$. Bei vollkommener farblicher Übereinstimmung zwischen dem Soll-Datensatz DS-S und dem Ist-Datensatz DS-I ist die Abweichung $\Delta E_{S,I}$ Null. Im Allgemeinen können jedoch auch andere Verfahren zum Berechnen der Abweichung $\Delta E_{S,I}$ herangezogen werden, wie beispielsweise auf der Basis von Spektralinformation.

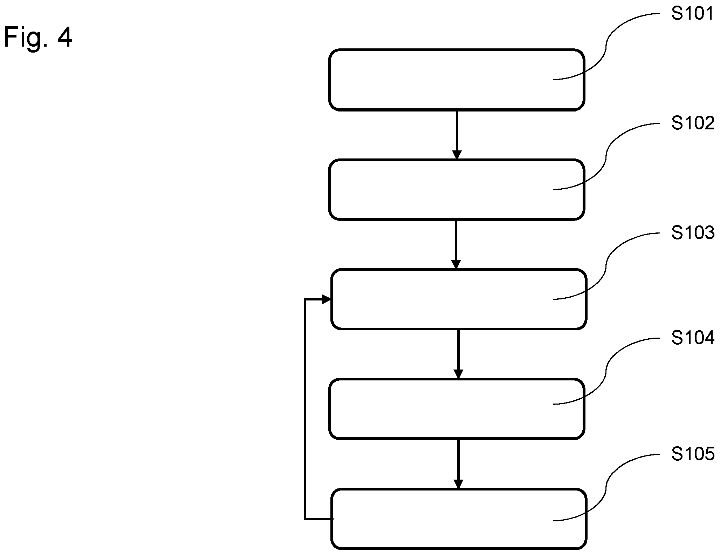

Fig. 4 zeigt ein Blockdiagram eines Verfahrens zum Gestalten einer dentalen Restauration 207. Im ersten Schritt S101 wird ein Soll-Datensatz DS-S auf Basis eines natürlichen Zahns 205 erfasst, der die optischen Eigenschaften, wie beispielsweise Farbe, Farbschema und Transluzenz, und/oder die Geometrie des natürlichen Zahns 205 wiedergibt. Das Erfassen des Solldatensatzes DS-S kann idealerweise an einem Nachbarzahn 205 des zu ersetzenden Zahns als Vorlage durchgeführt werden.

Dadurch wird der Vorteil erreicht, dass der Ist-Datensatz DS-I der dentalen Restauration 207 möglichst genau und farbgleich an die Mundsituation angepasst werden kann. Im Schritt S102 wird das digitale Zahnmodell 221 mit der inneren Architektur erzeugt, beispielsweise abgeleitet aus einem idealisierten Zahnmodell aus einer Zahnbibliothek, das als Grundlage für das weitere Verfahren dient. Im Schritt S103 werden dem digitalen Zahnmodell 221 die zu verwendenden Materialien und somit deren optische und physikalischen Parameter der inneren Architektur zugeordnet, die erforderlich sind, um das digitale Zahnmodell 221 zu rendern und daraus im nächsten Schritt den Ist-Datensatz DS-I zu erzeugen, der für den Soll/Ist-Vergleich herangezogen werden soll.

Anschließend wird in Schritt S104 die Abweichung $\Delta E_{S,I}$ zwischen dem Soll-Datensatz DS-S und dem Ist-Datensatz DS-I berechnet. Im Schritt S105 wird das digitale Zahnmodell 221 durch Zuordnung anderer Materialien und/oder Modifizierung der inneren Architektur so verändert, dass eine geringere Abweichung $\Delta E_{S,I}$ zwischen dem erfassten Soll-Datensatz DS-S und dem neu erzeugten Ist-Datensatz DS-I entsteht. Die Schritte S103 bis S105 werden iterativ wiederholt, bis die Abweichung $\Delta E_{S,I}$ zwischen dem Ist-Datensatz DS-I und dem Soll-Datensatz DS-S ein Minimum erreicht hat oder ein vorgegebenes Konvergenzintervall erreicht wird (Im Falle von Trade Offs z.B. Einhaltung minimaler Wandstärken, limitierte Verfügbarkeit von Dentalmaterialien, da eine Tendenz zur Reduktion besteht). Durch das Verfahren wird der technische Vorteil erreicht, dass die dentale Restauration 207 mit einem biomimetischen Aussehen hergestellt werden kann, das demjenigen des erfassten natürlichen Zahns 205 entspricht. Es besteht zudem der Vorteil, dass zukünftig mit einem geringeren Materialsortiment mehr Erscheinungsformen abgedeckt werden können.

Durch das iterative Verfahren zur Erzeugung von biomimetischen, dentalen Restaurationen 207 wird eine geschlossene Schleife realisiert, die solange durchlaufen wird, bis die Abweichung $\Delta E_{S,I}$ zwischen dem Ist-Datensatz DS-I und dem Soll-Datensatz DS-S am geringsten ist (Closed Loop). Dies betrifft beispielsweise die patientenspezifischen Farbmessung, die Präparation, die Modellierung, die Simulation und die Fertigung der hochästhetischen dentalen Restauration 207. Dabei handelt es sich um einen iterativen, geschlossenen digitalen Prozess, bei dem man sich durch die Modellierung und Simulation der dentalen Restauration 207 soweit wie möglich an das natürliche Erscheinungsbild im Patientenmund annähert. Die Verwendung von Farbschlüsseln jeglicher Art oder ein subjektiver Vergleich von Bildern ist überflüssig, da man sich an dem natürlichen Erscheinungsbild orientiert und ein automatisierter Abgleich erfolgt.

Bei dem Verfahren zum Gestalten der dentalen Restauration 207 handelt es sich um einen iterativen, geschlossenen digitalen Prozess, der die Modellierung und Simulation der dentalen Restauration 207 soweit wie möglich an das natürliche Erscheinungsbild im Patientenmund annähert. Dadurch dass die optischen Parameter der verwendeten Materialien bekannt sind und beim Rendern berücksichtigt werden, können die optischen Einflussfaktoren der unterschiedlichen dreidimensionalen Schichten, wie beispielsweise Präparation, Zementschicht, Dentin und Schneide, erstmals optisch realitätsnah simuliert werden, so dass sich ein realistischeres Erscheinungsbild für die eingesetzte dentalen Restauration 207 ergibt. Durch die Verwendung von Materialien mit bekannten optischen und physikalischen Eigenschaften (optisch kalibrierten Materialien) erkennt der Benutzer die biomimetische dentale Restauration 207 als realistisch.

Durch das Verfahren kann ein biomimetischer, d.h. der Natur sehr nahekommender, zahnähnlicher, geschichteter Aufbau der dentalen Restauration 207 bei den digitalen Fertigungsprozessen realisiert werden, wie Fräsen und 3D-Druck mit mehreren Materialien. Die Farbinformationen des Soll/Ist-Vergleichs können auch für die individuelle, selektive Einfärbung eines Zirkonoxid-Weißlings durch Infiltration verwendet werden. Das Verfahren betrifft den gesamten digitalen Workflow für indirekte dentale Restaurationen und bildet die Grundlage für die Umsetzung der Zahnästhetik einschließlich eines Multimaterial-3D-Drucks.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

**Patentansprüche**

1. Verfahren zum Gestalten einer dentalen Restauration (207), mit den Schritten:

   - Erfassen (S101) eines Soll-Datensatzes (DS-S) auf Basis eines natürlichen Zahns (205), der die optischen Eigenschaften und die Geometrie des natürlichen Zahns (205) wiedergibt;
   - Erzeugen (S102) eines digitalen Zahnmodells (221) mit einer inneren Architektur;
   - Rendern (S103) des digitalen Zahnmodells (221) auf Basis der inneren Architektur zum Erzeugen eines Ist-Datensatzes, der die optischen Eigenschaften und die Geometrie des digitalen Zahnmodells (221) wiedergibt;
   - Berechnen (S104) einer Abweichung ($\Delta E_{S,I}$) zwischen dem Soll-Datensatz (DS-S) und dem Ist-Datensatz (DS-I); und
   - Iteratives Verändern (S105) des digitalen Zahnmodells (221), um eine geringere Abweichung ($\Delta E_{S,I}$) zwischen dem erfassten Soll-Datensatz (DS-S) und dem Ist-Datensatz (DS-I) des erneut gerenderten digitalen Zahnmodells (221) zu erhalten; **dadurch gekennzeichnet, dass** die Abweichung ($\Delta ES,I$) auf Basis eines euklidischen Abstandes zwischen dem Soll-Datensatz (DS-S) und dem Ist-Datensatz (DS-I) berechnet wird oder die Abweichung ($\Delta ES,I$) auf Basis eines spektralen Abstandes zwischen dem Soll-Datensatz (DS-S) und dem Ist-Datensatz (DS-I) berechnet wird.

2. Verfahren nach einem der vorangehenden Ansprüche, wobei der Soll-Datensatz (DS-I) Daten über ein zweidimensionales oder dreidimensionales Abbild des natürlichen Zahns (205), Daten über einen Aufnahmewinkel zum Zahn (205), Daten über eine Farbinformation des Zahns (205), Daten über ein Farbspektrum des Zahns (205) und/oder Daten über eine Geometrieinformation des Zahns (205) umfasst.

3. Verfahren nach einem der vorangehenden Ansprüche, wobei der Soll-Datensatz (DS-S) durch eine digitale Kamera, einen 3D-Scanner, ein 3D-Kamerasystem, ein Spektrometer oder einen digitalisierten Farbschlüssel erfasst wird.

4. Verfahren nach einem der vorangehenden Ansprüche, wobei der Soll-Datensatz (DS-S) optische Eigenschaften eines Restzahns (209) umfasst.

5. Verfahren nach Anspruch 4, wobei das digitale Zahnmodell (221) auf Basis der optischen Eigenschaften des Restzahns (209), der Geometrie des Restzahns, der umgebenden Nachbarzähne, der Mundsituation, der Berücksichtigung der Gingiva oder Zahnfleischfarbe und/oder des verwendeten Klebematerials zur Fixierung der dentalen Restauration (207) auf dem Restzahn (209) gerendert wird.

6. Verfahren nach einem der vorangehenden Ansprüche, wobei der Schritt des Erzeugens des digitalen Zahnmodells (221) den Schritt eines Importierens, Berechnens und/oder Festlegens eines Zahnmodells (221) mit einer vorgegebenen inneren Architektur umfasst.

7. Verfahren nach einem der vorangehenden Ansprüche, wobei das digitale Zahnmodell (221) in einem Winkel gerendert wird, der dem Aufnahmewinkel beim Erfassen des Zahns (205) oder/und einer vorgegebenen Lichtsituation entspricht.

8. Verfahren nach einem der vorangehenden Ansprüche, wobei das digitale Zahnmodell (221) durch Änderung einer Materialzuordnung zu einem Subvolumen verändert wird.

9. Verfahren nach einem der vorangehenden Ansprüche, wobei das digitale Zahnmodell (221) durch eine Änderung des Subvolumens unter Beibehaltung der äußeren Geometrie verändert wird.

10. Verfahren nach einem der vorangehenden Ansprüche, wobei die dentale Restauration (207) auf Basis des Ist-Datensatzes (DS-I) hergestellt wird.

11. Verfahren nach Anspruch 10, wobei die Herstellung der dentalen Restauration (207) mittels einer Multimaterial-3D-Druckeinrichtung oder einer Fräseinrichtung erfolgt.

12. Computervorrichtung (200) zum Gestalten einer dentalen Restauration (207), mit einem Sensor (203) zum Erfassen eines Soll-Datensatzes auf Basis eines natürlichen Zahns, die geeignet ist, das Verfahren nach einem der Ansprüche 1 bis 11 auszuführen.

13. Computerprogramm, umfassend Befehle, die bewirken, dass die Computervorrichtung des Anspruchs 12 die Verfahrensschritte nach einem der Ansprüche 1 bis 11 ausführt.

**Claims**

1. A method for designing a dental restoration (207), comprising the steps of:

   - detecting (S101) a target data set (DS-S) based on a natural tooth (205) which reflects the optical properties and the geometry of the natural tooth (205);
   - generating (S102) a digital tooth model (221) with an internal architecture;
   - rendering (S103) the digital tooth model (221) based on the internal architecture to generate an actual data set which reflects the optical properties and the geometry of the digital tooth model (221);
   - calculating (S104) a deviation ($\Delta E_{S,I}$) between the target data set (DS-S) and the actual data set (DS-I); and
   - iterative altering (S105) the digital tooth model (221) to obtain a smaller deviation ($\Delta E_{S,I}$) between the detected target data set (DS-S) and the actual data set (DS-I) of the re-rendered digital tooth model (221),

   **characterized in that** the deviation ($\Delta E_{S,I}$) is calculated based on a Euclidean distance between the target data set (DS-S) and the actual data set (DS-I) or the deviation ($\Delta E_{S,I}$) is calculated based on a spectral distance between the target data set (DS-S) and the actual data set (DS-I).

2. The method according to any of the preceding claims,
   wherein the target data set (DS-I) comprises data about a two-dimensional or three-dimensional image of the natural tooth (205), data about an exposure angle to the tooth (205), data about color information of the tooth (205), data about a color spectrum of the tooth (205) and/or data about geometry information of the tooth (205).

3. The method according to any of the preceding claims,
   wherein the target data set (DS-S) is detected by a digital camera, a 3D scanner, a 3D camera system, a spectrometer, or a digitized color key.

4. The method according to any of the preceding claims,
   wherein the target data set (DS-S) comprises optical properties of a residual tooth (209).

5. The method according to claim 4, wherein the digital tooth model (221) is rendered based on the optical properties of the residual tooth (209), the geometry of the residual tooth, the surrounding neighboring teeth, the oral situation, the consideration of the gingiva or gingival color and/or the adhesive material used for fixing the dental restoration (207) on the residual tooth (209).

6. The method according to any of the preceding claims,
   wherein the step of generating the digital tooth model (221) comprises the step of importing, calculating and/or specifying a tooth model (221) having a predetermined internal architecture.

7. The method according to any of the preceding claims,
   wherein the digital tooth model (221) is rendered at an angle that corresponds to the exposure angle when detecting the tooth (205) and/or a predetermined light situation.

**8.** The method according to any of the preceding claims,
wherein the digital tooth model (221) is altered by altering a material assignment to a sub-volume.

**9.** The method according to any of the preceding claims,
wherein the digital tooth model (221) is altered by altering the sub-volume while retaining the outer geometry.

**10.** The method according to any of the preceding claims,
wherein the dental restoration (207) is produced based on the actual data set (DS-I).

**11.** The method according to claim 10, wherein the dental restoration (207) is produced by means of a multi-material 3D printing device or a milling device.

**12.** A computer device (200) for designing a dental restoration (207), comprising a sensor (203) for detecting a target data set based on a natural tooth, adapted to perform the method according to any of claims 1 to 11.

**13.** A computer program comprising instructions that cause the computer device of claim 12 to perform the method steps according to any of claims 1 to 11.

**Revendications**

**1.** Procédure de conception d'une restauration dentaire (207), avec les étapes suivantes :

- acquisition (S101) d'un jeu de données cibles (DS-S) sur la base d'une dent naturelle (205), qui reflète les propriétés optiques et la géométrie de la dent naturelle (205) ;
- génération (S102) d'un modèle numérique de dent (221) avec une architecture interne ;
- rendu (S103) du modèle numérique de dent (221) sur la base de l'architecture interne pour générer un jeu de données réelles qui reflète les propriétés optiques et la géométrie du modèle numérique de dent (221) ;
- calculer (S104) un écart ($\Delta E_{S,I}$) entre l'enregistrement cible (DS-S) et l'enregistrement réel (DS-I) ;
- modification itérative (S105) du modèle numérique de dent (221) pour obtenir un écart plus faible ($\Delta E_{s,I}$) entre le jeu de données cibles acquises (DS-S) et le jeu de données réelles (DS-I) du modèle numérique de dent (221) rendu à nouveau ;

**caractérisé en ce que** l'écart ($\Delta E_{S,I}$) est l'écart est calculé sur la base d'une distance euclidienne entre le jeu de données cibles (DS-S) et le jeu de données réelles (DS-I) ou l'écart ($AE_{S,I}$) est calculé sur la base d'une distance spectrale entre le jeu de données cible (DS-S) et le jeu de données réelles (DS-I).

**2.** Procédé selon l'une des revendications précédentes, où le jeu de données cible (DS-I) comprend des données sur une image bidimensionnelle ou tridimensionnelle de la dent naturelle (205), des données sur un angle de vue sur la dent (205), des données sur une information sur la couleur de la dent (205), des données sur un spectre de couleurs de la dent (205) et/ou des données sur une information géométrique de la dent (205).

**3.** Procédé selon l'une des revendications précédentes, où le jeu de données cibles (DS-S) est acquis par un appareil photo numérique, un scanner 3D, un système de caméra 3D, un spectromètre ou une clé de couleur numérisée.

**4.** Procédé selon l'une des revendications précédentes, où le jeu de données cible (DS-S) comprend les propriétés optiques d'une dent résiduelle (209).

**5.** Procédé selon la revendication 4, où le modèle numérique de dent (221) est rendu sur la base des propriétés optiques de la dent résiduelle (209), de la géométrie de la dent résiduelle, des dents adjacentes environnantes, de la situation buccale, de la prise en compte de la gencive ou de la couleur de la gencive et/ou du matériau adhésif utilisé pour fixer la restauration dentaire (207) sur la dent résiduelle (209).

**6.** Procédé selon l'une des revendications précédentes, où l'étape de génération du modèle numérique de dent (221) comprend l'étape d'importation, de calcul et/ou de spécification d'un modèle dentaire (221) avec une architecture interne donnée.

**7.** Procédé selon l'une des revendications précédentes, où le modèle numérique de dent (221) est rendu à un angle correspondant à l'angle de vue lorsque la dent est capturée (205) et/ou à une situation d'éclairage prédéfinie.

8. Procédé selon l'une des revendications précédentes, où le modèle numérique de dent (221) est modifié en un sous-volume en changeant l'affectation d'un matériau.

9. Procédé selon l'une des revendications précédentes, où le modèle numérique de dent (221) est modifié par une modification du sous-volume tout en conservant la géométrie externe.

10. Méthode selon l'une des revendications précédentes, où la restauration dentaire (207) est effectuée sur la base du jeu de données réelles (DS-I).

11. Procédé selon la revendication 10, où la restauration dentaire (207) est réalisée au moyen d'un dispositif d'impression 3D multi-matériaux ou d'un dispositif de fraisage.

12. Appareil informatique (200) pour la conception d'une restauration dentaire (207), comprenant un capteur (203) pour l'acquisition d'un jeu de données cibles sur la base d'une dent naturelle, qui convient à l'exécution de la méthode selon l'une des revendications 1 à 11.

13. Programme d'ordinateur comprenant des instructions qui permettent à l'appareil informatique de la revendication 12 d'exécuter les étapes du procédé conformément à l'une des revendications 1 à 11.

Fig. 1

EP 3 984 495 B1

Fig. 2

203

DS-S

$\Delta E_{S,I}$

Rendering

221

DS-I

Fig. 3

Fig. 4

S101

S102

S103

S104

S105

EP 3 984 495 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2486892 B1
- DE 19922870 A1
- WO 2019023461 A1